(19) Europäisches Patentamt / European Patent Office / Office européen des brevets

(11) **EP 1 582 165 B1**

(12) **EUROPEAN PATENT SPECIFICATION**

(45) Date of publication and mention
of the grant of the patent:
**08.07.2015 Bulletin 2015/28**

(51) Int Cl.:
*A61B 18/08* (2006.01)   *A61B 18/12* (2006.01)
*A61B 18/10* (2006.01)   *A61B 18/14* (2006.01)

(21) Application number: **05005029.3**

(22) Date of filing: **08.03.2005**

(54) **Operative instrument with heat-generating body**

Behandlungsinstrument mit Wärmeerzeuger

Dispositiv de traitement ayant un element thermique

(84) Designated Contracting States:
**DE FR GB**

(30) Priority: **12.03.2004   JP 2004071405**

(43) Date of publication of application:
**05.10.2005   Bulletin 2005/40**

(73) Proprietor: **Olympus Corporation
Tokyo 151-0072 (JP)**

(72) Inventors:
• **Kimura, Kenichi
Hachioji-shi
Tokyo (JP)**

• **Iida, Koji
Sagamihara-shi
Kanagawa (JP)**

(74) Representative: **von Hellfeld, Axel
Wuesthoff & Wuesthoff
Patentanwälte PartG mbB
Schweigerstrasse 2
81541 München (DE)**

(56) References cited:
WO-A-01/12090        WO-A-02/32333
WO-A-2004/002346     US-A- 4 031 898
US-A- 4 196 734      US-A1- 2003 171 747

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

CROSS-REFERENCE TO RELATED APPLICATION

This application is based upon and claims the benefit of priority from the prior Japanese Patent Application No. 2004-071405, filed March 12, 2004.

BACKGROUND OF THE INVENTION

1. Field of the Invention

The present invention relates to an operative instrument for heating an anatomy and coagulating or incising the same.

2. Description of the Related Art

A known operative instrument heats an anatomy for coagulating or incising the same. The known operative instruments include, for example, a heat treatment instrument, a high-frequency treatment instrument, and an ultrasonic treatment instrument.

The heat treatment instrument heats an anatomy clamped at the extremities of forceps provided with a heat-generating body, such as a heater, for coagulating or incising the same. The high-frequency treatment instrument clamps an anatomy between a pair of forceps and supplies a high-frequency current to the anatomy therebetween for heating and thereby coagulating or incising the same. The ultrasonic treatment instrument provides ultrasonic vibrations to the extremities of the forceps to generate frictional heat within an anatomy clamped therebetween for coagulating or incising the same.

In the related art, heat coagulation and incision forceps having a pair of grippers as described in Japanese Patent No. 3349139, or a treatment instrument provided with a heater wire at a working surface of a jaw member as described in WO01/12090 are proposed as the heat treatment instrument.

The heat coagulation and incision forceps stated in the above-described Patent No. 3349139 is provided with a heat-generating body on one of the grippers to coagulate and incise the gripped anatomy by causing the heat-generating body to generate heat.

The heat coagulation and incision forceps are controlled to maintain a constant temperature by a power supply unit so that the heat-generating body generates heat to keep the heat-generating body at a constant temperature. In the existing constant temperature control, electric power according to the temperature difference between the heat-generating body and a preset temperature is supplied to the heat-generating body. Since the initial temperature of the heat-generating body is low and hence the temperature difference with respect to the preset temperature is significantly large at the beginning of energization, large electric power is supplied to the heat-generating body to increase the temperature of the heat-generating body rapidly. On the other hand, when the temperature of the heat-generating body is increased to a value near the preset temperature, electric power lower than at the beginning of heating is supplied to the heat-generating body in order to prevent excessive heating of the heat-generating body, thereby lowering the heating rate, so that the preset temperature is achieved and maintained. In the existing constant temperature control, electric power supplied to the heat-generating body varies significantly from the beginning to the end of the heating process.

In the case of the heat coagulation and incision forceps, when the preset temperature of the heat-generating body is relatively high, the heat-generating body reaches rapidly a high temperature. Therefore, the period in which the temperature of the anatomy is in a range suitable for coagulation is short. Therefore, coagulation of the anatomy may be insufficient, and hence incision is performed at an early timing. In addition, when the preset temperature is relatively low, the heat coagulation and incision forceps needs a long time until the anatomy reaches a temperature suitable for incision. Consequently, while coagulation of the anatomy is done sufficiently, incision may be done relatively slowly.

The treatment instrument stated in the above described WO01/12090 employs the heat wire formed of an electric resistor, such as nichrome wire, as the heat-generating body, and is adapted to coagulate and incise the anatomy by heat generated by the heater wire.

Furthermore, an operative instrument stated in JP-A-10-286260 is used for treatment of an anatomy by heat generation. In this instrument, aerial heat dissipation preventing control is employed for preventing early deterioration of the heat-generating body.

Another example of a known operative instrument is described in the published United States Patent Application US 2003/171747. The operation of that instrument is not optimal, however.

BRIEF SUMMARY OF THE INVENTION

The present invention provides an operative instrument as recited in claim 1. Preferred features of the invention are recited in the dependent claims.

An operative instrument of the present invention includes a heat-generating body that generates heat to be applied to an anatomy in a treating section for treating the anatomy at an extremity of forceps. When heating the heat-generating body, a power supply unit supplies substantially constant electric power to the heat-generating body from the beginning of heating until the heat-generating body reaches a predetermined temperature. Since the heat-generating body is heated by substantially constant electric power from the beginning until it reaches the predetermined temperature, sufficient electric power for the anatomy to achieve a temperature for incising operation in a short period is supplied also after the anatomy is held at the temperature for the coagulating operation.

One of the methods for keeping electric power applied to the heat-generating body substantively constant is to keep electric voltage or electric current applied to the heat-generating body constant. Accordingly, even though the value of resistance of the heat-generating body varies to some extent due to rising of the temperature, the electric power applied to the heat-generating body is kept substantially constant.

One of the methods to keep electric power applied to the heat-generating body substantively constant is to control one or both of the applied electric voltage and the applied electric current to keep the applied electric power constant. In order to do so, values of voltage or current at the beginning of application may be determined by energizing the heat-generating body for monitoring at least when application is initiated.

Preferably, the value of resistance of the heat-generating body is used for monitoring the temperature. In general, when the temperature of the heat-generating body varies, the value of resistance thereof varies correspondingly. Therefore, the temperature of the heat-generating body can be detected by detecting changes in resistance value with the power supply unit. Accordingly, whether or not the heat-generating body reaches the predetermined temperature can be judged.

When the forceps do not grip the anatomy, the temperature of the heat-generating body rapidly increases. Therefore, when changes in voltage, current, or value of resistance within a given time period exceed a predetermined value, energization of the heat-generating body is stopped.

After the heat-generating body has reached the predetermined temperature, power supply to the heat-generating body may be stopped. Accordingly, the temperature of the heat-generating body is gradually lowered, but heating is started at a constant electric power by turning a switch on again.

After the heat-generating body has reached the predetermined temperature, the constant temperature control for keeping the temperature of the heat-generating body at the preset temperature may be performed.

After the heat-generating body has reached the predetermined temperature, the heat-generating body can be energized intermittently. In such intermittent energization, it is further preferable to apply constant voltage, current and electric power. In this arrangement, heat generation of the heat-generating body can be controlled by a duty cycle ratio control mode.

Preferably, when supplying substantially constant electric power to the power-generating body, the supplied electric power is gradually increased to the predetermined electric power for a short time immediately after energization. In this arrangement, heat distortion of the heat-generating body is decreased, thereby improving durability of the operative instrument.

Preferably, the heat-generating body is a heat-generating element having a thin film resistance or a thick film resistance having a temperature coefficient on the surface formed as a heat-generating pattern. In such an element, a change in electric resistance with respect to a change in temperature of its own are larger in comparison with the heater wire such as the nichrome wire. Therefore, the temperature can be detected easily from the resistance, and hence the temperature can be controlled easily.

BRIEF DESCRIPTION OF THE SEVERAL VIEWS OF THE DRAWINGS

These and other features, aspects, and advantages of the apparatus and methods of the present invention will become better understood with regard to the following description, appended claims, and accompanying drawings where:

Fig. 1 is a general block diagram showing an operative instrument according to a first embodiment;
Fig. 2 is a cross-sectional view taken along a line A-A in Fig. 1;
Fig. 3 is a cross-sectional view showing a modification of Fig. 2;
Fig. 4 is a drawing viewed in the direction indicated by an arrow B in Fig. 1;
Fig. 5 is a drawing viewed in the direction indicated by an arrow C in Fig. 1;
Fig. 6 is a perspective view of a heat-generating body;
Fig. 7 is a circuit block diagram of a power supply unit in Fig. 1;
Fig. 8 is a graph of output control performed by a control circuit in Fig. 7 showing output voltage with respect to time;
Fig. 9 is a graph of the output control performed by the control circuit in Fig. 7 showing output current with respect to time;
Fig. 10 is a graph of the output control performed by the control circuit in Fig. 7 showing output electric power with respect to time;
Fig. 11 is a graph of the output control performed by the control circuit in Fig. 7 showing electric resistance of the heat-generating body with respect to time;
Fig. 12 is a graph of the output control performed by the control circuit in Fig. 7 showing the temperature of an anatomy with respect to time;
Fig. 13 is a graph of a modification of the output control showing output voltage with respect to time;
Fig. 14 is a graph of a modification of the output control showing output current with respect to time;
Fig. 15 is a graph of a modification of the output control showing output electric power with respect to time;
Fig. 16 is a graph of a modification of the output control showing electric resistance of the heat-generating body with respect to time;

Fig. 17 is a graph of output control performed by a control circuit according to a second embodiment, and showing output voltage with respect to time;

Fig. 18 is a graph of the output control performed by the control circuit according to the second embodiment showing output current with respect to time;

Fig. 19 is a graph of the output control performed by the control circuit according to the second embodiment showing output electric power with respect to time;

Fig. 20 is a graph of the output control performed by the control circuit according to the second embodiment showing electric resistance of the heat-generating body with respect to time;

Fig. 21 is a graph of output control performed by the control circuit according to a third embodiment showing output voltage with respect to time;

Fig. 22 is a graph of the output control performed by the control circuit according to the third embodiment output current with respect to time;

Fig. 23 is a graph of the output control performed by the control circuit according to the third embodiment showing output electric power with respect to time;

Fig. 24 is a graph of the output control performed by the control circuit according to the third embodiment showing electric resistance of the heat-generating body with respect to time;

Fig. 25 is a graph of output voltage to the heat-generating body in a constant voltage control;

Fig. 26 is a graph of a first modification of Fig. 25;

Fig. 27 is a graph of a second modification of Fig. 25;

Fig. 28 is a graph of a voltage control with respect to time;

Fig. 29 is a graph of a first modification of Fig. 28;

Fig. 30 is a graph of a second modification of Fig. 28;

Fig. 31 is a graph of voltage control with respect to time when a thermal load is high;

Fig. 32 is a graph of voltage control with respect to time when the thermal load is low;

Fig. 33 is a graph of cumulative makeup heat quantity control with respect to time;

Fig. 34 is a graph of the temperature control of the heat-generating body or the anatomy with respect to time; and

Fig. 35 is a graph of voltage control with respect to time.

## DETAILED DESCRIPTION OF PREFERRED EMBODIMENTS OF THE INVENTION

Preferred embodiments of the invention will be described below with reference to the accompanying drawings.

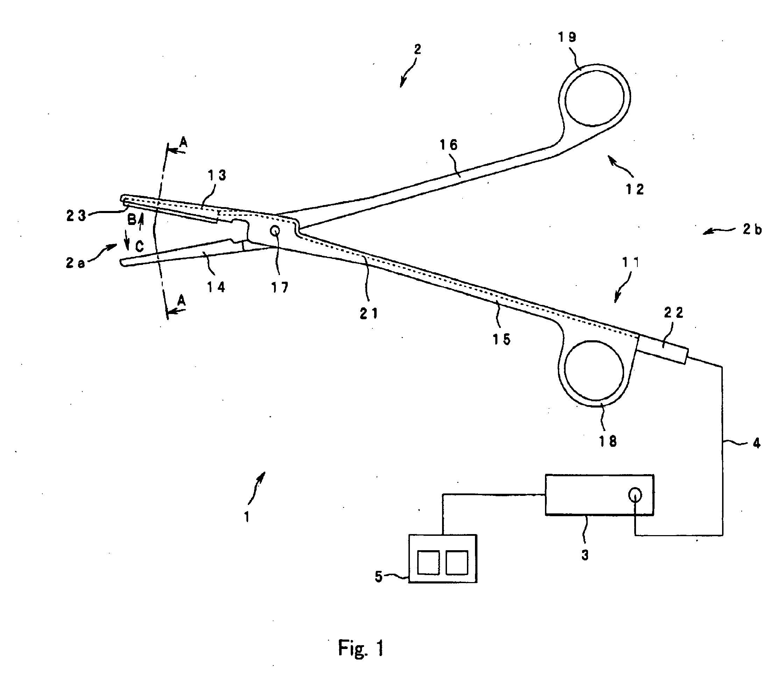

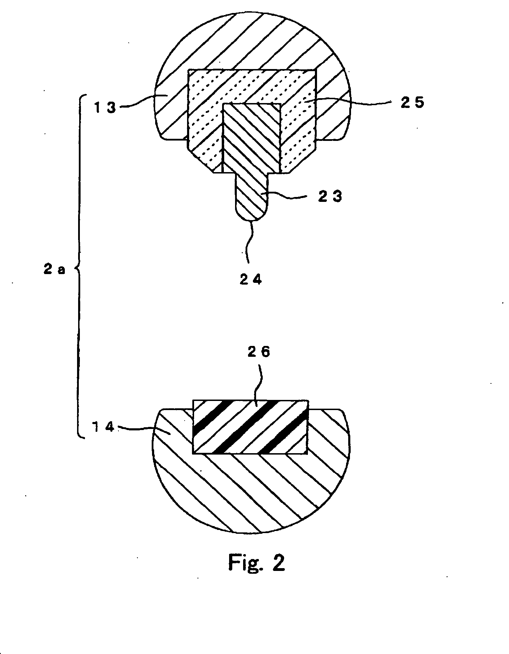

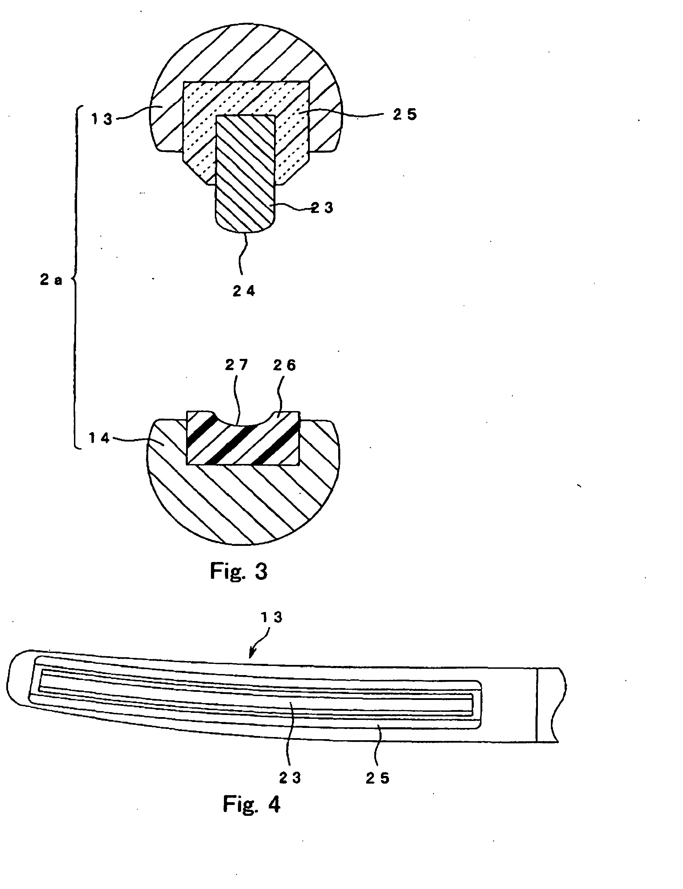

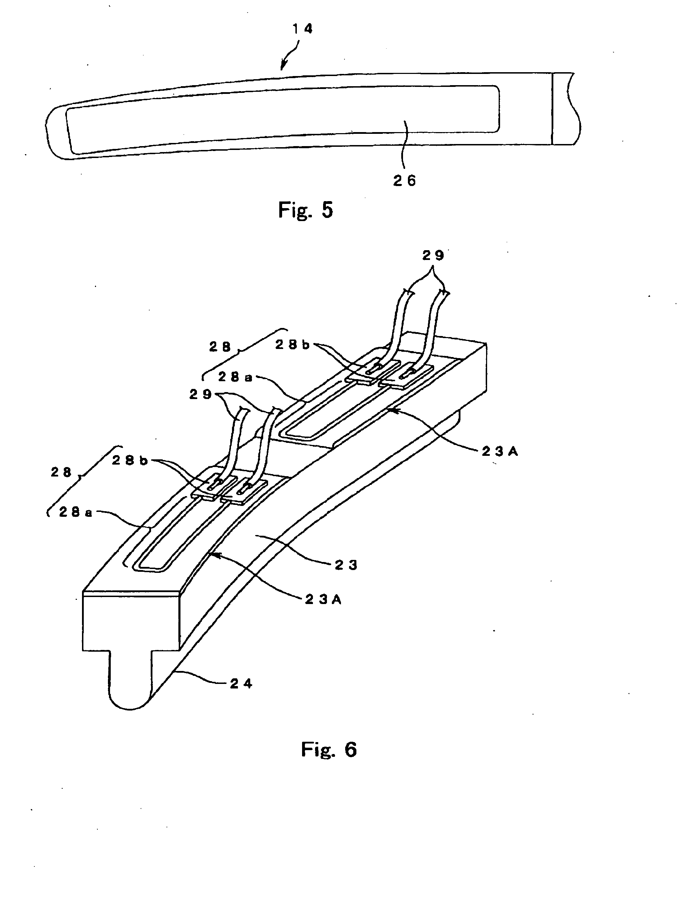

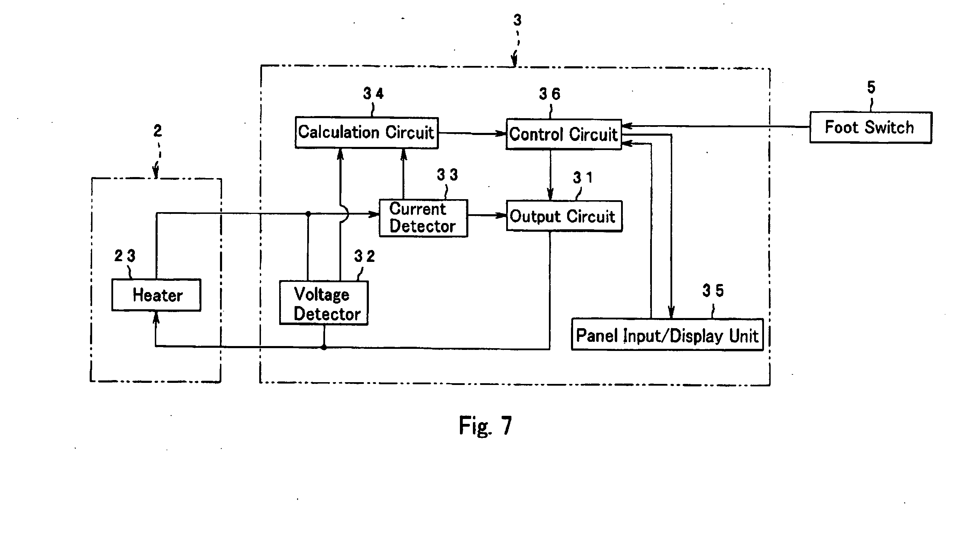

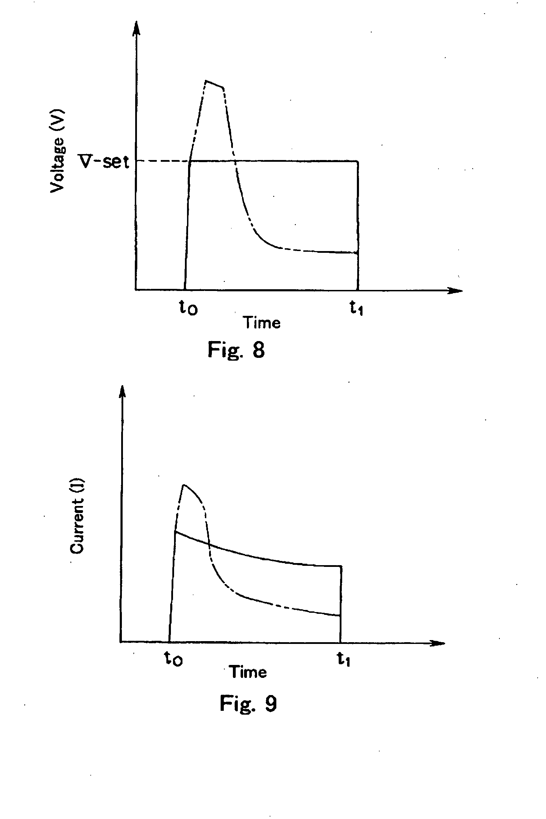

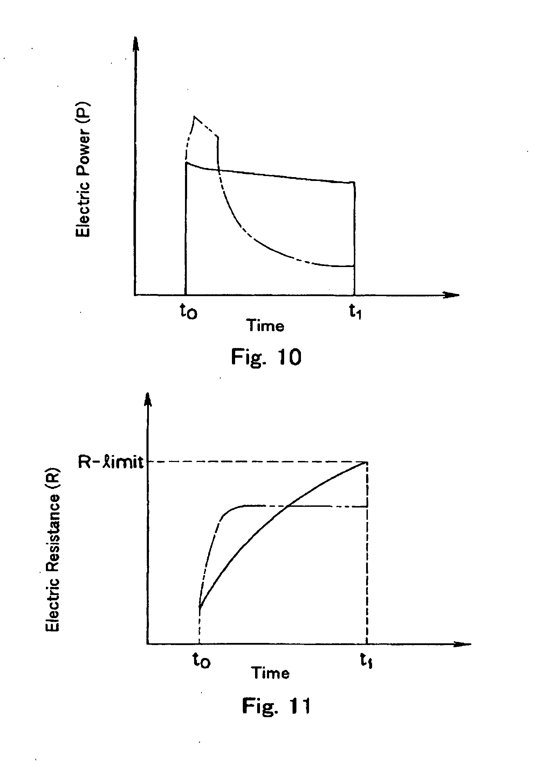

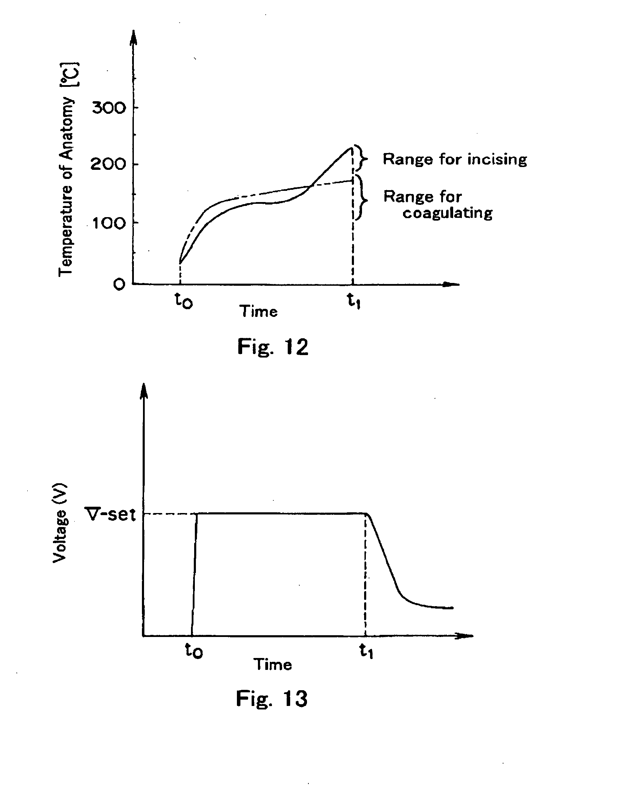

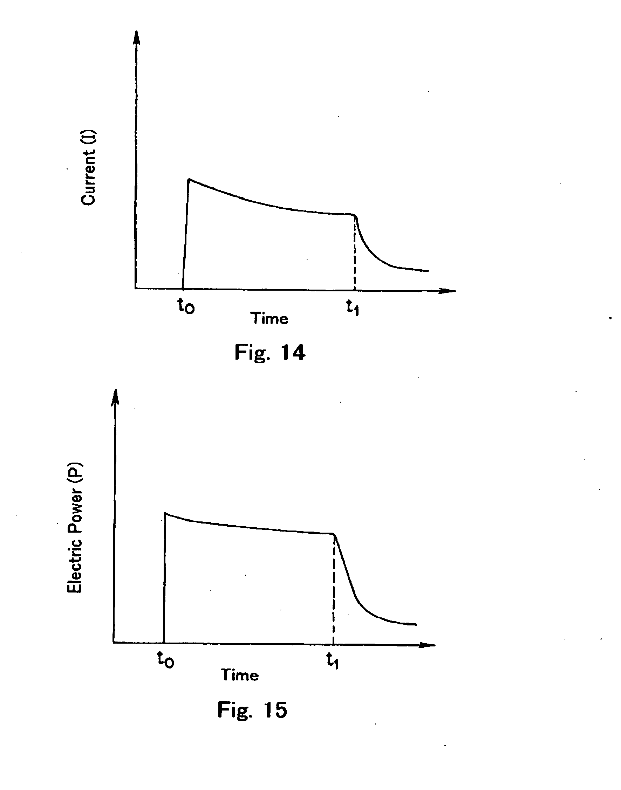

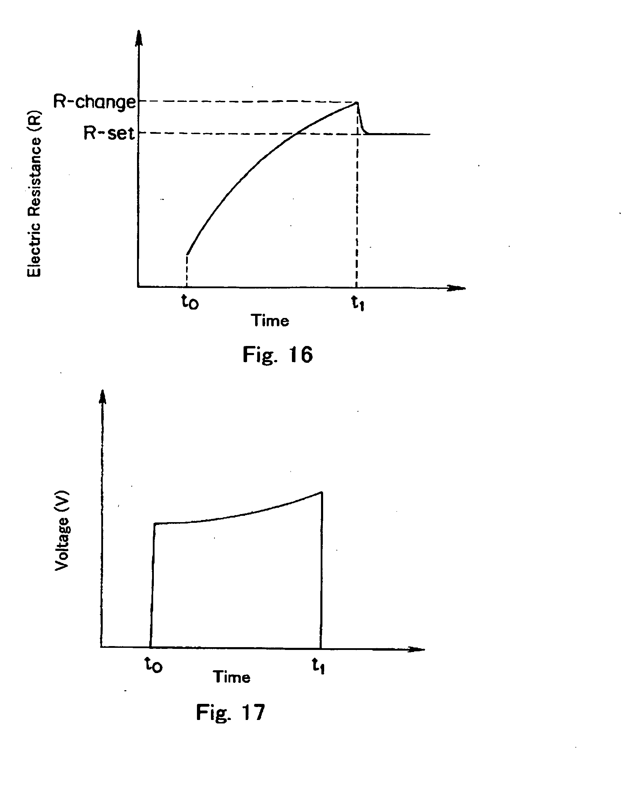

Fig. 1 to Fig. 16 relate to a first embodiment of the invention. Fig. 1 is a general block diagram showing an operative instrument according to the first embodiment; Fig. 2 is a cross-sectional view taken along a line A-A in Fig. 1; Fig. 3 is a cross-sectional view showing a modification of Fig. 2; Fig. 4 is a drawing viewed in the direction indicated by an arrow B in Fig. 1; Fig. 5 is a drawing viewed in the direction indicated by an arrow C in Fig. 1; and Fig. 6 is a perspective view of a heat-generating body. Fig. 7 is a circuit block diagram of a power supply unit in Fig. 1 and Fig. 8 to Fig. 12 are graphs showing an output control performed by a control circuit in Fig. 7. Fig. 8 is a graph showing an output voltage with respect to time. Fig. 9 is a graph showing an output current with respect to time; Fig. 10 is a graph showing an output electric power with respect to time; Fig. 11 is a graph showing an electric resistance of the heat-generating body with respect to time; and Fig. 12 is a graph showing the temperature of an anatomy with respect to time. Fig. 13 to Fig. 16 are graphs showing modifications of the output control. Fig. 13 is a graph showing an output voltage with respect to time; Fig. 14 is a graph showing an output current with respect to time; Fig. 15 is a graph showing an output electric power with respect to time; and Fig. 16 is a graph showing an electric resistance of the heat-generating body with respect to time.

As shown in Fig. 1, an operative instrument 1 of the first embodiment includes forceps 2 that provide heat to an anatomy which they grip to effect coagulating and incising that anatomy. A power supply unit 3 that can be disconnectably connected to the forceps 2 outputs electric power of a power source (electric energy) to the forceps 2 to drive and control the same.

The forceps 2 are adapted in such a manner that a connector (not shown) provided at the rear end of a connecting cord 4 extending therefrom can be disconnectably connected to the power supply unit 3.

A foot switch 5 can be connected to the power supply unit 3. The operator can turn the power source to the forceps 2 ON and OFF by turning the foot switch 5 ON and OFF. A front panel of the power supply unit 3 is provided with a display device for displaying a current value or a voltage value of the power source supplied to the forceps 2 and an operating tab for entering various setting values on a panel input/display unit, described later.

The forceps 2 mainly include a pair of handles 11, 12 to be held and operated by the operator's hand, a pair of jaws 13, 14 for gripping an anatomy to be treated for coagulation and incision, and a pair of pincers members 15, 16.

The jaws 13, 14 constitute a treating section 2a of the forceps 2.

The pair of pincers members 15, 16 extend between the handles 11, 12 and the jaws 13, 14, respectively. The pincers members 15, 16 are overlapped substantially in a state of crossing at the midsections thereof. Furthermore, the crossed portion of the pincers members 15, 16 is provided with a pivot pin 17 for rotatably joining the pincers members 15, 16.

The handles 11, 12 are provided with finger insertion rings 18, 19. The forceps 2 are so constructed that back and forth movement of a thumb and an annular finger inserted into the respective rings 18, 19 causes the jaws 13, 14 to open and close correspondingly, so that they can grip, separate, and pressurize the anatomy. In other words, the pair of handles 11, 12 and the pair of pincers members 15, 16 constitute an operating section 2b of the forceps 2.

The jaw 13 includes a heat-generating body, described later, embedded therein for providing heat to the anatomy. A power supply line 21 for supplying electric power to the heat-generating body is provided within the pincers member 15. In the present embodiment, a heat-generating element having a thin film resistance or a thick film resistance on the surface thereof as a heat-generating pattern is employed, as described later.

The power supply line 21 extends from the jaw 13 to the handle 11, and is adapted to be electrically connected to the power supply unit 3 from a cord connecting portion 22 on the rear side of the ring 18 via the connecting cord 4 to the power supply unit 3.

The operative instrument 1 is configured in such a manner that, after the forceps 2 have griped the anatomy via the treating section 2a, electric power is supplied from the power supply unit 3 to the heat-generating body to generate heat, and the heat is provided to the gripped anatomy for coagulation and incision.

Below are detailed descriptions of the structures of the respective portions of the operative instrument 1.

The treating section 2a includes, as shown in Fig. 2, the jaw 13 provided with a heat-generating body 23 for providing heat to the anatomy at a position opposed to the jaw 14. The heat-generating body 23 is formed of a material having high heat conductivity, such as copper or molybdenum. The heat-generating body 23 is formed with a tissue pressing portion 24 having a relatively dull or coarse shape on the surface facing the jaw 14.

The upper side of the heat-generating body 23 is covered by a heat-insulating member 25 formed of a material having low heat conductivity and high heat resistance, such as PTFE (polyfluoroethylene, or polytetrafluoroethylene) or PEEK (polyetheretherketone).

The heat-insulating member 25 is fixed to the jaw 13 by being fitted in a recess of the jaw 13. Accordingly, the heat-generating body 23 provides generated heat efficiently to the anatomy, and prevents the jaw 13 formed of metal material such as stainless from becoming excessively heated.

The heat-generating body 23 is provided with coating formed of non-adhesive material such as PTFE, not shown, at the contact area with respect to the anatomy for preventing attachment of the anatomy thereto.

On the other hand, the jaw 14 is integrally provided with a receiving member 26 at the position opposed to the heat-generating body 23. The receiving member 26 is formed of resin material such as silicon rubber or PTFE.

The treating section 2a may have a modified structure as shown in Fig. 3.

As shown in Fig. 3, the jaw 13 is formed with the tissue pressing portion 24 of the heat-generating body 23 formed into a shape which is more dull or coarse than the one shown in Fig. 2. The receiving member 26 of the jaw 14 is formed with a recess 27 that engages the tissue pressing portion 24 of the heat-generating body 23.

In addition to those shown in Fig. 2 and Fig. 3, other combinations of the heat-generating body 23 and the receiving member 26 of various shapes and materials may be employed. Also, as shown in Fig. 4 and Fig. 5, the jaw 13 and the jaw 14 are curved along their length dimensions and tapered toward the distal ends thereof.

As shown in Fig. 6, the upper surface of the heat-generating body 23 provided on the jaw 13 is formed with a thin film substrate 23A formed with a pattern 28 of a resistance heat-generating body by means of a thin film forming method (such as PVD: Physical Vapor Deposition or CVD :Chemical Vapor Deposition), or a thick film forming method (screen printing).

The pattern 28 includes a heat-generating area 28a that generates heat by being energized and a lead wire mounting portion 28b which is a non-heat-generating area. The pattern 28 is formed of high-melting point metal such as molybdenum, which increases in electric resistance in proportion to the temperature, that is, which has a so-called positive temperature coefficient.

The power supply line 21 disposed within the pincers member 15 is provided with a lead wire 29 for supplying electric power to the pattern 28. The distal end of the lead wire 29 is connected to the lead wire mounting portion 28b of the pattern 28 by soldering or by thermocompression bonding. In the present embodiment, two of the patterns 28 are formed on the heat-generating body 23, and the two patterns 28 are electrically connected to the power supply unit 3 respectively so that output control can be made independently.

Referring now to Fig. 7, the internal structure of the power supply unit 3 will be described.

The power supply unit 3 includes an output circuit 31 for supplying electric power for causing the heat-generating body 23 to generate heat, a voltage detecting unit 32 for detecting voltage applied to the heat-generating body 23 (pattern 28), a current detecting unit 33 for detecting a current flowing in the heat-generating body 23, a calculating circuit 34 for calculating various parameters such as voltage, current, electric power, and value of resistance, a panel input/display unit 35 for displaying a current value or a voltage value of the power source supplied to the heat-generating body 23 or entering various preset values, and a control circuit 36 for controlling the output circuit 31 based on the result of calculation made by the calculation circuit 34 according to the various preset values preset by the panel input/display unit 35.

The foot switch 5 is connected to the control circuit 36, and ON and OFF of the output circuit 31 is controlled in accordance with ON and OFF of the foot switch 5.

When the control circuit 36 controls ON and OFF operation of the output circuit 31 in accordance with positions of the foot switch 5 and controls the output circuit 31 by comparing various preset values entered via the panel input/display unit 35 and respective parameters (voltage V, current I, electric power P, electric resistance R) as results of calculation by the calculation circuit 34, output control, described later, is provided for the heat-generating body 23 (pattern 28).

The operative instrument 1 in this arrangement is controlled as shown by the graphs in Fig. 8 to Fig. 12. First, the operator enters a preset voltage V-set and an upper limit temperature T-limit through the panel input/display unit 35.

Then the operator holds the forceps 2, positions the anatomy between the jaws 13, 14 and, in this state, operates the operating section 2b to the closing direction, so that the anatomy is clamped and gripped between the heat-generating body 23 and the receiving member 26. Subsequently, the operator turns the foot switch 5 to ON.

Electric power is supplied to the heat-generating body 23 of the forceps 2 from the power supply unit 3 via the connecting cord 4, the cord connecting portion 22, and the lead wire 29, and the heat-generating body 23 generates heat.

Here, changes in voltage V, current I, electric power P, and electric resistance R and a change in temperature of the anatomy in constant temperature control in the related art are shown by double-dashed line in Fig 8 to Fig 12. In the constant temperature control in the related art, electric power supplied immediately after starting output is large, but electric power supplied after the heat-generating body reaches the preset temperature drops significantly.

Therefore, in the constant temperature control in the related art, insufficient electric power is supplied to the heat-generating body after the anatomy reaches the temperature for coagulating operation, and hence it takes long time until the anatomy reaches the temperature for incision operation. In other words, in the constant temperature control in the related art, coagulation is performed sufficiently, but incision is performed slowly.

If the preset temperature of the heat-generating body is increased in order to perform incision quickly, the anatomy cannot be held at the temperature for coagulating operation for a sufficient time period, and reaches too rapidly the temperature for incision operation. Therefore, coagulation of the anatomy cannot be performed properly. In other words, in the constant temperature control in the related art, it is difficult to perform incision quickly in a state in which the anatomy is sufficiently coagulated.

In contrast, according to the present embodiment, control is performed in such a manner that the voltage V to be applied to the heat-generating body 23 is kept constant (constant voltage control), and output from the output circuit 31 is stopped when the heat-generating body 23 reaches the preset upper limit temperature T-limit. In other words, by turning the foot switch 5 ON (time t0 in Fig. 8 to Fig. 12), the preset voltage V-set is applied to the heat-generating body 23 of the forceps 2, as shown in Fig. 8. The heat-generating body 23 starts heat generation and increases in temperature, and the electric resistance R of the pattern 28 increases as shown in Fig. 11.

When the electric resistance R of the pattern 28 reaches a preset threshold R-limit (a value of resistance of the pattern 28 at the upper limit temperature T-limit) (time t1 in Fig. 8 to Fig. 12), the control circuit 36 stops output from the output circuit 31. The control circuit 36 converts the threshold R-limit of the pattern 28 from T-limit.

Accordingly, the temperature of the heat-generating body 23 does not exceed the preset upper limit temperature T-limit. As shown in Fig. 9, the current I flowing to the heat-generating body 23 out of electric power output from the power supply unit 3 is gradually reduced.

Therefore, the electric power P supplied to the heat-generating body 23 is reduced with time although it is just a small amount. However, the substantially constant electric power P is supplied to the heat-generating body 23.

Therefore, as shown in Fig. 10, since sufficient electric power P is supplied to the heat-generating body 23 after the anatomy is kept at the temperature for coagulating operation, the anatomy reaches the temperature for incision operation in a short time. Accordingly, the forceps 2 can perform incision quickly while keeping a sufficient coagulating capacity.

The temperature of the anatomy increases with initiation of heat generation of the heat-generating body 23 as shown in Fig. 12. Subsequently, the rate of increase in temperature of the anatomy in the vicinity of about 100 to 150 °C is lower. This is because the electric power P supplied to the heat-generating body 23 is consumed as energy that vaporizes water contained in the anatomy. During this period, sufficient coagulating operation takes place in the anatomy. Upon completion of evaporation of water contained in the anatomy, the temperature of the anatomy starts increasing again. When the anatomy reaches the temperature for incising operation (about 200°C), incision of the tissue is performed.

Accordingly, the operative instrument 1 can perform incision quickly in a state in which the anatomy is sufficiently coagulated. Therefore, the time required for the entire operation is reduced. Also, since the operative instrument 1 uses the heat-generating body 23 (pattern 28) having a positive temperature coefficient, it can control the temperature more precisely.

In addition, since the temperature of the heat-generating body of the operative instrument 1 does not increase excessively, it can be used repeatedly. Accordingly, reduction of the cost of the operative instrument 1 is realized.

During the constant temperature control shown in Fig. 8 to Fig. 12, when the power supply unit 3 starts power output in a state in which the forceps 2 are not gripping the anatomy, the temperature of the heat-generating body 23 of the operative instrument 1 rapidly increases, and hence the current I flowing in the heat-generating body 23 is rapidly reduced.

Therefore, the control circuit 36 may be adapted in such a manner that the amount of change in current per unit time $\Delta t$, $|\Delta I \div \Delta t|$ is calculated by the calculating circuit 34, and the output is stopped when the amount of change in current $|\Delta I \div \Delta t|$ exceeds the preset value. Here, the delta $\Delta$ represents a difference.

Accordingly, since the operative instrument 1 can prevent output from the power supply unit 3 in a state in which the forceps 2 are not gripping the anatomy, safety during treatment is improved. In addition, the operative instrument 1 can also prevent a rapid change in temperature of the heat-generating body 23.

The operative instrument 1 can perform the constant temperature control as shown in Fig. 13 to Fig. 16.

In this modification, the voltage V applied to the heat-generating body 23 is kept constant (constant voltage control), in which the heat-generating body 23 is kept at a constant preset temperature T-set after the heat-generating body 23 reaches a predetermined changing temperature T-change.

In other words, in Fig. 13 to Fig. 16, the control circuit 36 operates in such a manner that the pattern 28 is kept at a constant electric resistance R-set (a value of resistance of the pattern 28 at the preset temperature T-set) after the electric resistance R of the pattern 28 reaches a threshold R-change (a value of resistance of the pattern 28 at the changing temperature T-change) (time period t1 in Fig. 13 to Fig. 16).

In this case as well, the operative instrument 1 can perform quick incision while keeping a sufficient coagulating capacity as in the case of the output control described in conjunction with Fig. 8 to Fig. 12.

When performing control according to the present modification, it is necessary for the operator to enter and set the preset voltage V-set, the changing temperature T-change, and the preset temperature T-set via the panel input/display unit 35 in advance. In addition to the example shown in Fig. 13 to Fig. 16, the relation between the changing temperature T-change and the preset temperature T-set may either be T-change = T-set or T-change < T-set.

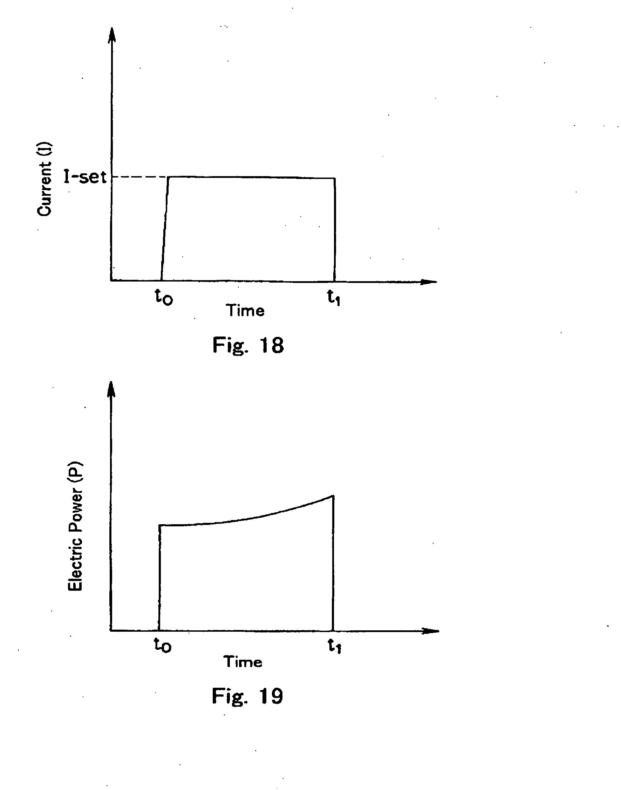

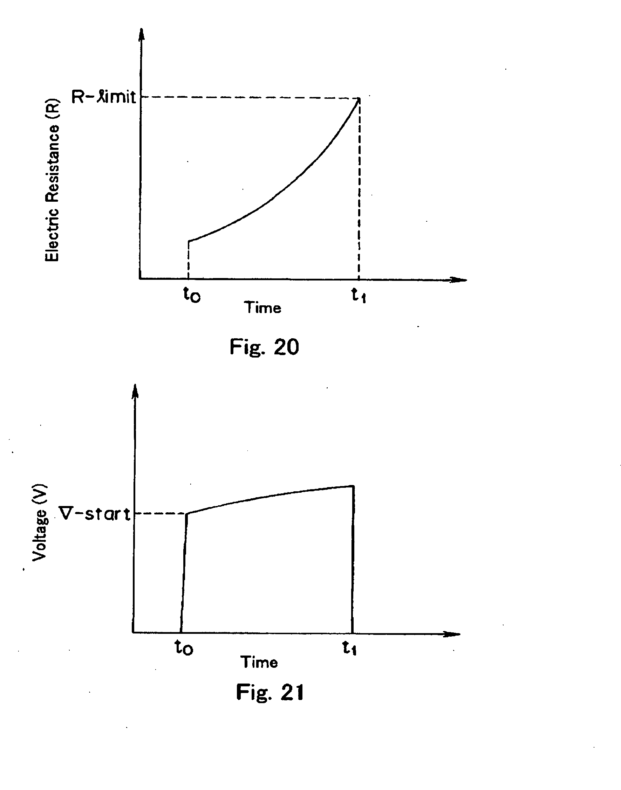

Fig. 17 to Fig. 20 is a graph of output control performed by the control circuit according to a second embodiment of the invention. Fig. 17 is a graph of output voltage with respect to time; Fig. 18 is a graph of output current with respect to time; Fig. 19 is a graph of output electric power with respect to time; and Fig. 20 is a graph of electric resistance of the heat-generating body with respect to time.

Although constant voltage control is performed in the first embodiment, constant current control is performed in the second embodiment. Other structures are the same as the first embodiment and hence will not be described again, and the same structures are represented by the same reference numeral in description.

In other words, as shown in Fig. 17 to Fig. 20, the operative instrument 1 of the second embodiment is controlled in such a manner that the current I flowing in the heat-generating body 23 is kept constant (constant current control), and output is stopped when the heat-generating body 23 reaches the upper limit temperature T-limit.

Thus, the control circuit 36 is configured to flow a preset current I-set to the heat-generating body 23 by turning the foot switch 5 ON (time to in Fig. 17 to Fig. 20), so that the heat-generating body 23 starts generating heat and hence the temperature increases. As the temperature of the heat-generating body 23 increases, the electric resistance R of the pattern 28 increases as well.

The control circuit 36 stops the output from the output circuit 31 when the electric resistance R of the pattern 28 reaches the preset threshold R-limit (a value of resistance of the pattern 28 at the upper limit temperature T-limit) (time t1 in Fig. 17 to Fig. 20). Accordingly, the temperature of the heat-generating body 23 does not exceed the preset upper limit temperature T-limit. During output, the voltage V applied to the heat-generating body 23 gradually increases.

Consequently, the electric power P supplied to the heat-generating body 23 increases with time although it is just a small amount. Therefore, substantially constant electric power P is supplied to the heat-generating body 23. A change in temperature of the anatomy is the same as in Fig. 12 described relative to the first embodiment.

Therefore, with the operative instrument 1, since the anatomy is kept at the temperature for coagulating operation and then a sufficient electric power P is supplied to the heat-generating body 23, the anatomy reaches the temperature of incision operation in a short time. Accordingly, the operative instrument 1 can perform quick incision while keeping a sufficient coagulating capacity. When performing control according to the second embodiment, it is necessary for the operator to enter and set the preset current I-set and the upper limit temperature T-limit via the panel input/display unit 35 in advance.

During constant current control as in Fig. 9, it is also possible to control the operation in such a manner that the amount of change in voltage per unit time $\Delta t$, $|\Delta V \div \Delta t|$ is calculated by the calculating circuit 34, and the output is stopped when the amount of change in voltage $|\Delta V \div \Delta t|$ xceeds the preset value.

Accordingly, the second embodiment can prevent power output in a state in which the forceps 2 are not gripping the anatomy as in the first embodiment, safety during treatment is improved. In addition, the operative instrument 1 according to the second embodiment prevents rapid changes in temperature of the heat-generating body 23.

Also, in the second embodiment, in the same manner as with the first embodiment described above, it is also possible to input the preset current I-set, the changing temperature T-change, and the preset temperature T-set via the panel input/display unit 35, and then perform constant current control followed by constant temperature control.

Therefore, the operative instrument 1 of the second embodiment can achieve the same effect as the first embodiment.

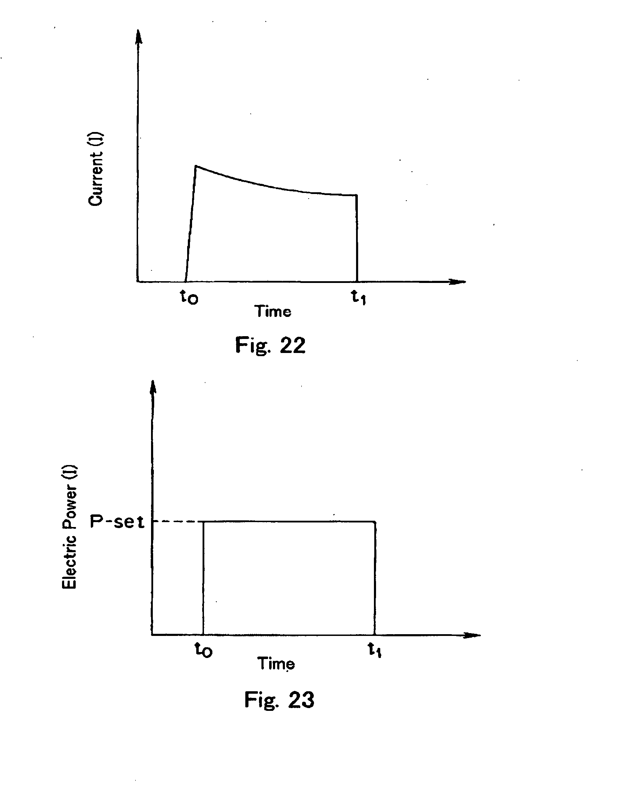

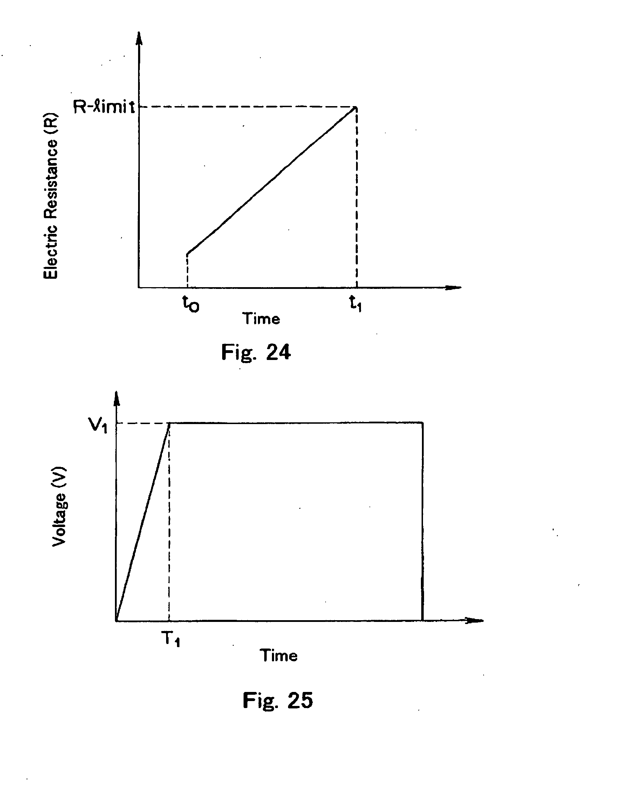

Fig. 21 to Fig. 24 are graphs of output control performed by a control circuit according to a third embodiment of the invention. Fig. 21 is a graph showing output voltage with respect to time; Fig. 22 is a graph of output current with respect to time; Fig. 23 is a graph of output electric power with respect to time; and Fig. 24 is a graph of electric resistance of the heat-generating body with respect to time.

While constant voltage control is provided in the first embodiment, the third embodiment is based on constant electric power control. Since other structures are the same as in the first embodiment, description will not be made again and the same structures are represented by the same reference numerals in description.

In other words, in the operative instrument 1 according to the third embodiment, the electric power P supplied to the heat-generating body 23 is kept constant (constant electric power control), and output is stopped when the heat-generating body 23 reaches the preset upper limit temperature T-limit as shown in Fig. 21 to Fig. 24.

Thus, in the control circuit 36, a preset electric power P-set is supplied to the heat-generating body 23 by turning the foot switch 5 ON (time to in Fig. 21 to Fig. 24), and the heat-generating body 23 starts generating heat and hence the temperature increases. The electric resistance R of the pattern 28 increases with increase in temperature of the heat-generating body 23.

Here, the control circuit 36 stops output from the output circuit 31 when the electric resistance R of the pattern 28 reaches the preset threshold R-limit (value of resistance of the pattern at the upper limit temperature T-limit) (time t1 in Fig. 21 to Fig. 24).

Accordingly, the temperature of the heat-generating body 23 does not exceed the preset upper limit temperature T-limit. The electric power P supplied to the heat-generating body 23 is constant during output.

Therefore, in the operative instrument 1, since a sufficient electric power P is supplied to the heat-generating body 23 after the anatomy is kept at the temperature for coagulating operation, the anatomy reaches the temperature for incising operation in a short time.

Accordingly, the operative instrument 1 can perform quick incision while keeping a sufficient coagulating capacity.

When performing the third embodiment, it is necessary for the operator to enter and set the preset electric power P-set, and the upper temperature T-limit via the panel input/display unit 35 in advance. A change in temperature of the anatomy is the same as in Fig. 12 described in the first embodiment.

When the foot switch 5 is turned ON, an extremely low monitoring current I-m flows in the heat-generating body 23 for an extremely short time (on the order of 0.1 second). Here, a starting voltage value V-start at the beginning of output is calculated by the calculating circuit 34. The expression of the starting voltage value V-start is as follows.

$$V\text{-start} = (P\text{-set} * V\text{-m} / I\text{-m})^{1/2}$$

Where, V-m represents voltage to be applied to the heat-generating body 23 for supplying the monitoring current I-m.

In the operative instrument 1, constant electric power control by the control circuit 36 is started by applying the starting voltage value V-start.

Accordingly, in the operative instrument 1 according to the third embodiment, an excessive voltage V is not applied to the heat-generating body 23 when starting the power output.

The operative instrument 1 according to the third embodiment may be configured in such a manner that the monitoring current I-m is constantly supplied and constant electric power control is started immediately after the foot switch 5 is turned ON.

In the operative instrument 1 according to the third embodiment, during constant electric power control as shown in Fig. 21 to Fig. 24, it is also possible to operate in such a manner that the amount of change in resistance per unit time $|\Delta R \div \Delta t|$ is calculated by the calculating circuit 34, and the output is stopped when the amount of change in resistance $|\Delta R \div \Delta t|$ exceeds the preset value.

Accordingly, in the operative instrument 1 according to the third embodiment, since output in a state in which the forceps are not gripping the anatomy can be prevented as in the first embodiment, safety during treatment is improved. In addition, the operative instrument 1 according to the third embodiment prevents rapid changes in temperature of the heat-generating body 23.

In the operative instrument 1 according to the third embodiment, as in the case of the first embodiment described above, it is also possible to input the preset electric power P-set, the changing temperature T-change, and the preset temperature T-set via the panel input/display unit 35, and then perform constant electric power control followed by constant temperature control.

Preferably, the operative instrument is controlled in such a manner that output voltage immediately after starting heat generation is gradually increased during constant voltage control of the heat-generating body.

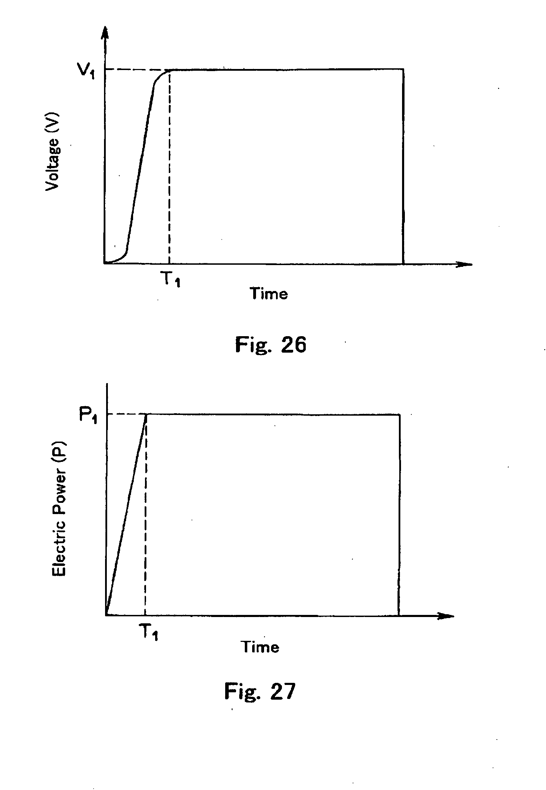

Fig. 25 to Fig. 27 are graphs in which output voltage and output electric power immediately after starting heat generation are controlled. Fig. 25 is a graph of output voltage to the heat-generating body in the constant voltage control; Fig. 26 is a graph of a first modification of Fig. 25; and Fig. 27 is a graph of a second modification of Fig. 25.

As shown in Fig. 25, the operative instrument is configured in such a manner that voltage gradually increases for a predetermined time period T1 immediately after starting output and reaches a predetermined voltage V1 after T1 has elapsed in the constant voltage control (T1 is on the order of 1ms to 1s).

Consequently, by increasing voltage gradually for an extremely short time immediately after starting output, the temperature of the heat-generating body of the operative instrument does not increase excessively, and thermal distortion is reduced. Accordingly, durability of the heat-generating body of the operative instrument is improved.

As shown in Fig. 26, it is also possible to control the operative instrument by further dividing change in voltage immediately after starting output timewise.

In other words, as shown in Fig. 26, the operative instrument further divides change in voltage immediately after starting output timewise, so that it changes smoothly immediately after starting output and when reaching the predetermined voltage V1.

Also, as shown in Fig. 27, the operative instrument may be controlled by changing output electric power immediately after starting output.

As shown in Fig. 27, in the operative instrument, output electric power immediately after starting output is changed in the constant electric power control. In the case of the constant electric power control shown in Fig. 27, it is also possible to control by further dividing change in electric power during output as in the case of the constant voltage control in Fig. 25. Although not shown, it is also possible to control in such a manner that output current immediately after starting output is gradually increased in the constant current control.

Also, preferably, the operative instrument is configured to increase the temperature of the heat-generating body efficiently by performing constant voltage control when outputting electric power, and then to perform control of applying voltage in pulses.

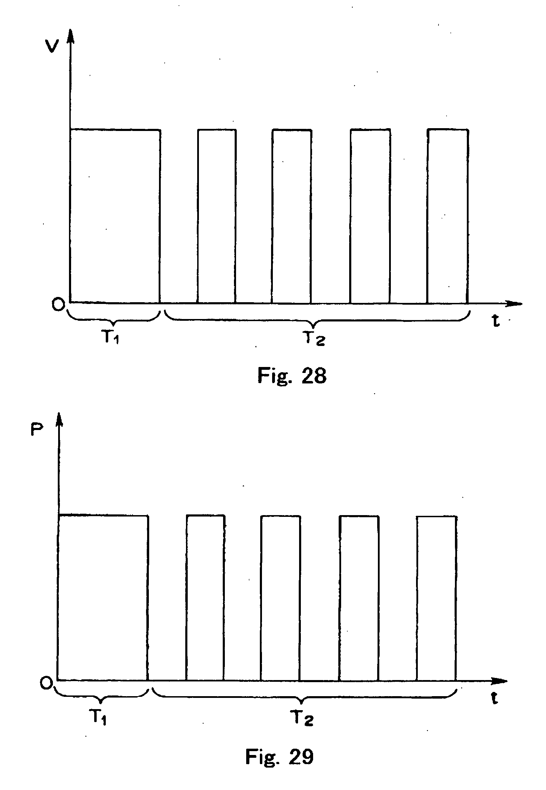

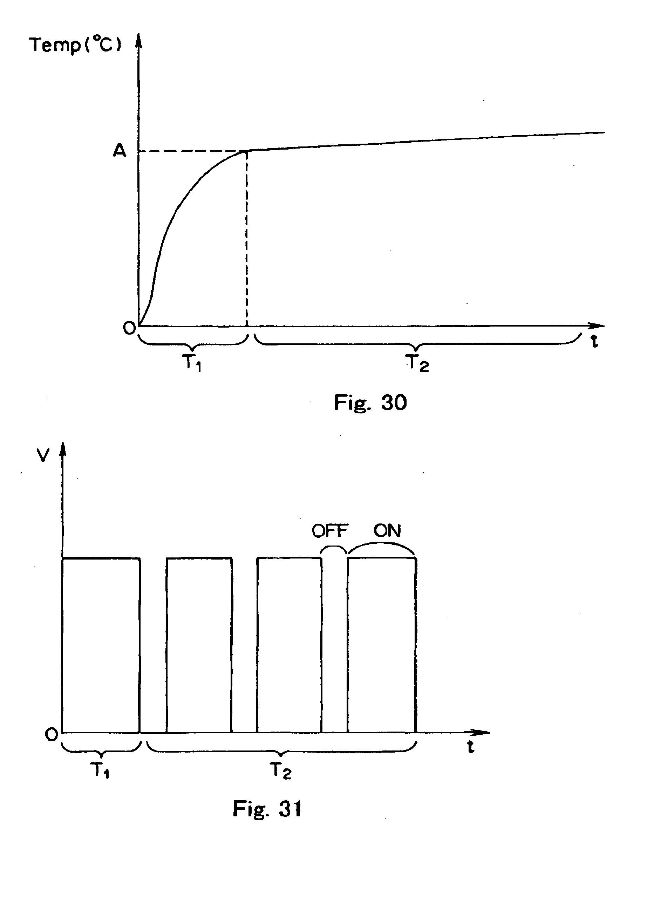

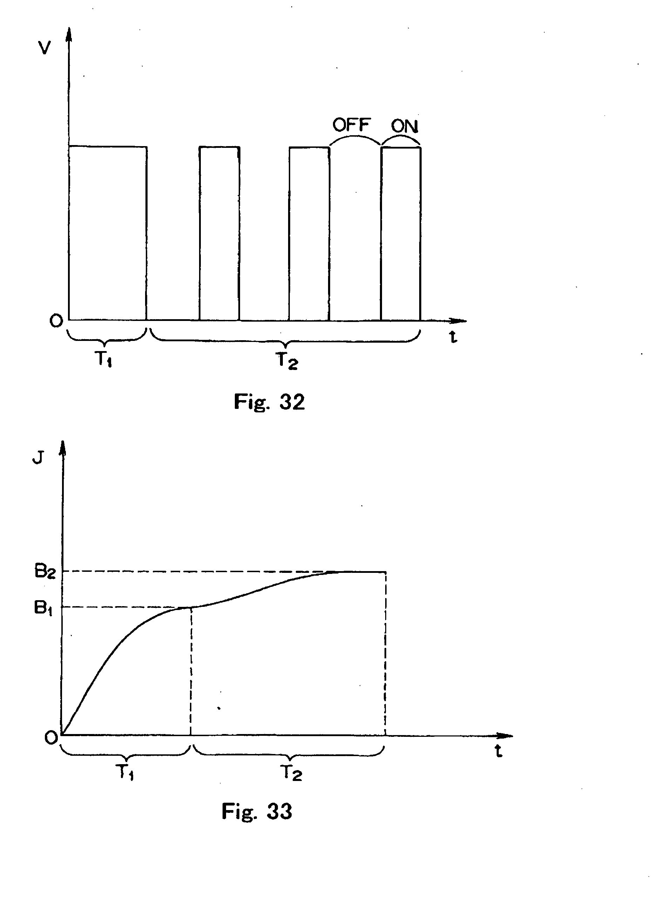

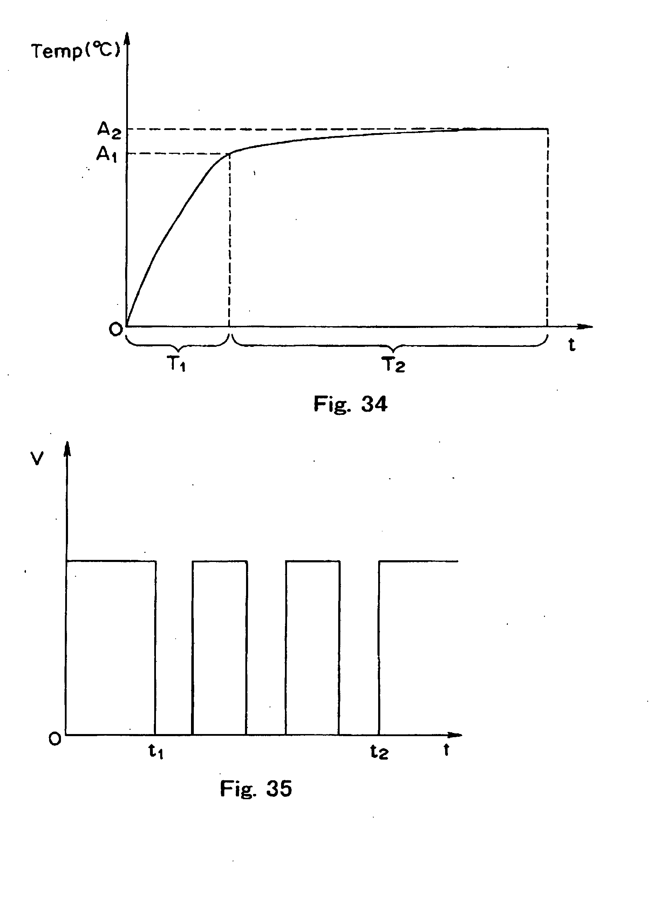

Fig. 28 to Fig. 35 are graphs of electric power supply control in which constant voltage control is performed when outputting electric power to efficiently increase the temperature of the heat-generating body, by applying voltage in pulses. Fig. 28 is a graph of voltage control with respect to time. Fig. 29 is a graph of a first modification of Fig. 28; Fig. 30 is a graph of a second modification of Fig. 28; Fig. 31 is a graph of voltage control with respect to time when a thermal load is high; and Fig. 32 is a graph of voltage control with respect to time when the thermal load is low. Fig. 33 is a graph of cumulative makeup heat quantity control with respect to time; Fig. 34 is a graph of the temperature control of the heat-generating body or the anatomy with respect to time; and Fig. 35 is a graph of voltage control with respect to time.

As shown in Fig. 28, the operative instrument is driven by constant voltage control for the time period T1 after the power supply unit has started outputting and increase the temperature of the heat-generating body.

After the given time period T1 has elapsed for the time period T2, control is switched to pulse control and energy to be supplied to the heat-generating body is reduced by constant voltage control.

Accordingly, the operative instrument raises the temperature of the anatomy modestly or gradually, and hence carbonization of the anatomy is prevented, so that reliable coagulation is ensured. The parameter of output may be electric power control as shown in Fig. 29.

Switching from constant voltage control to pulse control may be achieved by providing a function for monitoring the temperature of the heat-generating body or the temperature of the anatomy to the power source and switching output control of the power source from constant voltage control to pulse control at a time when the temperature reaches a given threshold A as shown in Fig 30. Although it is not shown in the drawing, the threshold may be not only the temperature, but also the time period from the moment when output is started, or the value of the cumulative makeup heat quantity from the moment when output is started.

Accordingly, since the heat-generating body reaches the temperature suitable for treating the anatomy immediately after starting output, the operative instrument can achieve a desired temperature rapidly by constant voltage or constant electric power control. After having reached the desired temperature, the operative instrument outputs power by pulse control for preventing excessive heating of the anatomy, and hence reliable coagulation is achieved while minimizing heat supply to the anatomy.

Consequently, the operative instrument can achieve reliable coagulation without causing carbonization of the anatomy.

The thermal load applied to the heat-generating body differs depending on the structure of the treatment instrument or the state of the tissue. Therefore, the ratio between ON-OFF durations of pulse control (referred to as Duty ratio) may not be an optimal setting when treatment of different anatomies is performed with a single setting.

Therefore, the operative instrument is configured so that the operator can select Duty ratio by setting of the power source depending on the magnitude of the thermal load applied to the heat-generating body.

In other words, the operative instrument is configured in such a manner that the ratio of ON duration of Duty ratio is increased when the thermal load is high as shown in Fig. 31, and the ratio of ON duration is decreased when the thermal load is low as shown in Fig. 32 by the selection of setting of the power source by the operator.

Accordingly, the operative instrument can select an optimal setting depending on the anatomy or the treatment instrument, and hence reliable coagulation of the anatomy is possible without causing carbonization.

The operative instrument may be configured in such a manner that a function for monitoring cumulative heat quantity to be outputted to the anatomy (not shown) is provided to the power supply unit.

In this case, the output is stopped at the moment when the cumulative heat quantity exceeds a given threshold B2 as shown in Fig. 33. It is also possible to stop outputting at the moment when a given time period T2 has elapsed after a given cumulative heat quantity B1 has exceeded and hence control is converted into pulse control. In the same manner, as shown in Fig. 34, thresholds A1, A2 may be provided in temperature of the heat-generating body or the temperature of the anatomy.

Accordingly, the operative instrument can stop output from the power supply unit automatically, and hence reliable coagulation is achieved without causing carbonization of the anatomy.

As shown in Fig. 35, the operative instrument can be configured by combining controls from Fig. 28 to Fig. 34.

In other words, as shown in Fig. 35, the operative instrument increases the temperature of the heat-generating body by constant voltage control from the moment when output is started to a given timing t1. The operative instrument converts control from constant voltage control to pulse control when the given timing t1 has elapsed.

At this moment, the timing t1 may be set by providing a threshold to the temperature of the heat-generating body or the anatomy as described in conjunction with Fig. 30, or by providing a threshold to the cumulative makeup heat quantity as described in conjunction with Fig. 33. Alternatively, it is also possible to enable setting of the timing t1 in advance.

The pulse control is adapted in such a manner that the operator can select Duty ratio as described in conjunction with Fig. 31 and Fig. 32.

The operative instrument is adapted to continue pulse control after the pulse control is stared until a given timing t2, and then after the timing t2, to switch the constant voltage control again, so that incision of the anatomy is performed in association with increase in temperature of the heat-generating body. Setting of the timing t2 may be performed by providing a threshold to the temperature or the makeup heat quantity as in the case of the timing t1, or may be set in advance. The parameter of output may be voltage, current, or electric power.

The operative instrument switches control of constant voltage, pulse, and constant voltage automatically, and can perform treatment to carry out raising the temperature of the heat-generating body, coagulating the anatomy, and incising the anatomy in ordered fashion.

Accordingly, the operative instrument can achieve reliable coagulation and incision simultaneously while minimizing thermal damage of the anatomy.

While there has been shown and described what are considered to be preferred embodiments of the invention, it will, of course, be understood that various modifications and changes in form or detail could readily be made without departing from the invention. It is therefore intended that the invention be not limited to the exact forms described and illustrated, but should be construed to cover all modifications that may fall within the scope of the appended claims.

**Claims**

1. An operative instrument (1) comprising:

   forceps (2) having a treating section (2a) for treating an anatomy held at an extremity thereof and including a heat-generating body (23) for generating heat to be provided to the anatomy for coagulating and incising same, and
   a power supply unit (3) that provides electrical energy to the heat-generating body (23), **characterized in that** the power supply unit (3) is adapted to supply substantially constant electric power to the heat-generating body (23) of the forceps (2) to increase the temperature of the heat-generating body to a predetermined temperature, wherein the power supply unit (3) is adapted to stop energization of the heat-generating body (23) when a change of voltage or current supplied, or a value of resistance of the heat-generating body (23) with time exceeds a predetermined value.

2. An operative instrument (1) according to claim 1, wherein the power supply unit (3) is adapted to supply the substantially constant electric power to the heat-generating body (23) during a period immediately after starting electric power supply to the heat-generating body until the temperature of the heat-generating body rises to the predetermined temperature.

3. An operative instrument (1) according to claim 1, wherein the power supply unit (3) is adapted to supply the substantially constant electric power to the heat-generating body (23) during a period from a time when a predetermined time period has elapsed after having started electric power supply to the heat generating body until a time when the temperature of the heat-generating body reaches the predetermined temperature.

4. An operative instrument (1) according to claim 1, wherein the power supply unit (3) is controlled so that one of constant voltage or constant current is supplied during a period in which the substantially constant electric power is supplied to the heat-generating body (23).

5. An operative instrument (1) according to claim 1, wherein the power supply unit (3) is adapted to detect the temperature of the heat-generating body (23) from a value of resistance of the heat-generating body.

6. An operative instrument (1) according to claim 1, wherein the power supply unit (3) is adapted to control the heat-generating body at a constant temperature after the temperature of the heat-generating body (23) reaches the predetermined temperature.

7. An operative instrument (1) according to claim 1, wherein the power supply unit (3) is adapted to apply electric power to the heat-generating body (23) intermittently after the temperature of the heat-generating body reaches the predetermined temperature.

8. An operative instrument (1) according to claim 1, wherein the heat-generating body (23) is a heat-generating element comprising a thin film resistance or a thick film resistance having a temperature coefficient as a heat-generating pattern on the surface thereof.

9. An operative instrument (1) according to claim 1, wherein the heat-generating body (23) has a positive temperature coefficient, and wherein the power supply unit (3) is constructed to calculate the temperature of the heat-generating body from the electric resistance of the heat-generating body and, when the electric resistance of the heat-generating body reaches a predetermined value, to determine that the temperature of the heat-generating body has reached the predetermined temperature and to stop electric power supply to the heat-generating body or switch control to constant temperature control which keeps the temperature of the heat-generating body at the predetermined temperature.

10. An operative instrument (1) according to claim 1, wherein the power supply unit is adapted to determine that the forceps (2) are not treating the anatomy when the amount of change of one of voltage to be applied to the heat-generating body, current to be supplied to the heat-generating body, and electric resistance of the heat-generating body exceeds a predetermined value, and to stop electric power supply to the heat-generating body.

11. An operative instrument (1) according to claim 1, wherein the power supply unit (3) supplies a monitoring current to the heat-generating body (23) before starting output to the heat-generating body or after starting output, calculates a voltage value to be applied to the heat-generating body based on voltage and current values of the monitoring current and a preset value of electric power, and starts electric power supply to the heat-generating body based on the calculated voltage value.

12. An operative instrument (1) according to claim 1, wherein the forceps (2) comprises a pair of grippers (13, 14) capable of opening and closing the treating section (2a) on the distal side and an operating section (2b) for opening and closing the grippers (13, 14) on the proximal side, and wherein the heat-generating body (23) is provided on at least one of the grippers (13, 14) of the forceps.

13. An operative instrument (1) according to claim 1, wherein the power supply unit (3) comprises a calculating circuit (34) for supplying a monitoring current to the heat generating body (23) before starting output to the heat-generating body or after starting output, calculating a voltage value to be applied to the heat-generating body (23) based on a voltage and current values of the monitoring current and a preset value of electric power, and wherein the operative instrument starts electric power supply to the heat-generating body (23) based on the calculated voltage value calculated by the calculating circuit (34).

**Patentansprüche**

1. Betriebsinstrument (1) umfassend:

   eine Zange (2) mit einem Behandlungsabschnitt (2a) zum Behandeln einer Anatomie, die an einer Extremität davon gehalten wird, und aufweisend einen wärmeerzeugenden Körper (23) zum Erzeugen von Wärme, die an der Anatomie zum Koagulieren und Einschneiden derselben bereitgestellt wird, und

   eine Leistungsversorgungseinheit (3), die elektrische Energie an dem wärmeerzeugenden Körper (23) bereitstellt, **dadurch gekennzeichnet, dass** die Leistungsversorgungseinheit (3) dazu ausgebildet ist, im Wesentlichen konstante elektrische Leistung an den wärmeerzeugenden Körper (23) der Zange (2) zu liefern, um die Temperatur des wärmeerzeugenden Körpers auf eine vorbestimmte Temperatur zu erhöhen, wobei die Leistungsversorgungseinheit (3) dazu ausgebildet ist, Bestromung des wärmeerzeugenden Körpers (23) zu stoppen, wenn eine Änderung einer gelieferten Spannung oder eines gelieferten Stroms oder ein Widerstandswert des wärmeerzeugenden Körpers (23) über der Zeit einen vorbestimmten Wert übersteigt.

2. Betriebsinstrument (1) nach Anspruch 1, wobei die Leistungsversorgungseinheit (3) dazu ausgebildet ist, die im Wesentlichen konstante elektrische Leistung an den wärmeerzeugenden Körper (23) während einer Zeitspanne zu liefern sofort nach Starten der elektrischen Leistungsversorgung an den wärmeerzeugenden Körper bis die Temperatur des wärmeerzeugenden Körpers auf die vorbestimmte Temperatur steigt.

3. Betriebsinstrument (1) nach Anspruch 1, wobei die Leistungsversorgungseinheit (3) dazu ausgebildet ist, die im Wesentlichen konstante elektrische Leistung an den wärmeerzeugenden Körper (23) während einer Zeitspanne zu liefern von einer Zeit, wenn eine vorbestimmte Zeitspanne abgelaufen ist nachdem die elektrische Leistungsversorgung an den wärmeerzeugenden Körper gestartet ist, bis zu einer Zeit, wenn die Temperatur des wärmeerzeugenden Körpers die vorbestimmte Temperatur erreicht.

4. Betriebsinstrument (1) nach Anspruch 1, wobei die Leistungsversorgungseinheit (3) derart gesteuert wird, dass eine konstante Spannung oder ein konstanter Strom während einer Zeitspanne geliefert wird, in welcher die im Wesentlichen konstante elektrische Leistung an den wärmeerzeugenden Körper (23) geliefert wird.

5. Betriebsinstrument (1) nach Anspruch 1, wobei die Leistungsversorgungseinheit (3) dazu ausgebildet ist, die Temperatur des wärmeerzeugenden Körpers (23) von einem Widerstandswert des wärmeerzeugenden Körpers zu erfassen.

6. Betriebsinstrument (1) nach Anspruch 1, wobei die Leistungsversorgungseinheit (3) dazu ausgebildet ist, den wärmeerzeugenden Körper bei einer konstanten Temperatur zu steuern, nachdem die Temperatur des wärmeerzeugenden Körpers (23) die vorbestimmte Temperatur erreicht.

7. Betriebsinstrument (1) nach Anspruch 1, wobei die Leistungsversorgungseinheit (3) dazu ausgebildet ist, elektrische Leistung in Abständen an den wärmeerzeugenden Körper (23) anzulegen, nachdem die Temperatur des wärmeerzeugenden Körpers die vorbestimmte Temperatur erreicht.

8. Betriebsinstrument (1) nach Anspruch 1, wobei der wärmeerzeugende Körper (23) ein wärmeerzeugendes Element ist, umfassend einen Dünnschichtwiderstand oder einen Dickschichtwiderstand mit einem Temperaturkoeffizienten als ein wärmeerzeugendes Muster auf der Oberfläche davon.

9. Betriebsinstrument (1) nach Anspruch 1, wobei der wärmeerzeugende Körper (23) einen positiven Temperaturkoeffizienten hat und wobei die Leistungsversorgungseinheit (3) derart gebildet ist, die Temperatur des wärmeerzeugenden Körpers aus dem elektrischen Widerstand des wärmeerzeugenden Körpers zu berechnen und, wenn der elektrische Widerstand des wärmeerzeugenden Körpers einen vorbestimmten Wert erreicht, zu bestimmen, dass die Temperatur des wärmeerzeugenden Körpers die vorbestimmte Temperatur erreicht hat, und die elektrische Leistungsversorgung an den wärmeerzeugenden Körper zu stoppen oder die Steuerung auf eine konstante Temperatursteuerung zu schalten, welche die Temperatur des wärmeerzeugenden Körpers bei der vorbestimmten Temperatur hält.

10. Betriebsinstrument (1) nach Anspruch 1, wobei die Leistungsversorgungseinheit dazu ausgebildet ist, zu bestimmen, dass die Zangen (2) nicht die Anatomie behandeln, wenn die Menge einer Änderung einer an dem wärmeerzeugenden Körper anzulegenden Spannung, eines an den wärmeerzeugenden Körper zu liefernden Stroms, oder eines elektrischen Widerstands des wärmeerzeugenden Körpers einen vorbestimmten Wert übersteigt, und die elektrische Leistungsversorgung an den wärmeerzeugenden Körper zu stoppen.

11. Betriebsinstrument (1) nach Anspruch 1, wobei die Leistungsversorgungseinheit (3) einen Beobachtungsstrom an den wärmeerzeugenden Körper (23) liefert bevor die Ausgabe an den wärmeerzeugenden Körper gestartet wurde oder nachdem die Ausgabe gestartet wurde, einen an den wärmeerzeugenden Körper anzulegenden Spannungswert basierend auf Spannungs- und Stromwerten des Beobachtungsstroms und einem vorgegebenen Wert der elektrischen Leistung berechnet, und elektrische Leistungsversorgung an den wärmeerzeugenden Körper basierend auf dem berechneten Spannungswert startet.

12. Betriebsinstrument (1) nach Anspruch 1, wobei die Zangen (2) ein Paar von Greifern (13, 14) umfassen, die zum Öffnen und Schließen des Behandlungsabschnitts (2a) an dem distalen Ende fähig sind, und einen Betriebsabschnitt (2b) zum Öffnen und Schließen der Greifer (13, 14) auf der proximalen Seite, und wobei der wärmeerzeugende Körper (23) an zumindest einem der Greifer (13, 14) der Zangen vorgesehen ist.

13. Betriebsinstrument (1) nach Anspruch 1, wobei die Leistungsversorgungseinheit (3) eine Berechnungsschaltung (34) umfasst zum Liefern eines Beobachtungsstroms an den wärmeerzeugenden Körper (23) vor dem Starten der Ausgabe an den wärmeerzeugenden Körper oder nach dem Starten der Ausgabe, zum Berechnen eines an den wärmeerzeugenden Körper (23) anzulegenden Spannungswerts basierend auf Spannungs- und Stromwerten des Beobachtungsstroms und einem vorgegebenen Wert der elektrischen Leistung, und wobei das Betriebsinstrument eine elektrische Leistungsversorgung an den wärmeerzeugenden Körper (23) basierend auf dem berechneten Spannungswert startet, der durch die Berechnungsschaltung (34) berechnet wurde.

## Revendications

1. Instrument opératoire (1) comprenant :

    une pince (2) ayant une section (2a) de traitement pour traiter une anatomie maintenue à une extrémité de celle-ci et incluant un corps (23) de génération de chaleur pour générer une chaleur destinée à être délivrée à l'anatomie pour coaguler et inciser celle-ci, et
    une unité (3) d'alimentation électrique qui délivre une énergie électrique au corps (23) de génération de chaleur, **caractérisé en ce que** l'unité (3) d'alimentation électrique est adaptée à délivrer une puissance électrique sensiblement constante au corps (23) de génération de chaleur de la pince (2) pour élever la température du corps de génération de chaleur jusqu'à une température prédéterminée, dans lequel l'unité (3) d'alimentation électrique est adaptée à stopper la mise sous tension du corps (23) de génération de chaleur lorsqu'un changement de tension ou de courant délivré(e), ou une valeur de résistance du corps (23) de génération de chaleur avec le temps excède une valeur prédéterminée.

2. Instrument opératoire (1) selon la revendication 1, dans lequel l'unité (3) d'alimentation électrique est adaptée à délivrer la puissance électrique sensiblement constante au corps (23) de génération de chaleur lors d'une période immédiatement après le début de l'alimentation électrique du corps de génération de chaleur jusqu'à ce que la température du corps de génération de chaleur s'élève jusqu'à la température prédéterminée.

3. Instrument opératoire (1) selon la revendication 1, dans lequel l'unité (3) d'alimentation électrique est adaptée à délivrer la puissance électrique sensiblement constante au corps (23) de génération de chaleur lors d'une période d'un moment où une durée de temps prédéterminée s'est écoulée après avoir commencé l'alimentation électrique du corps de génération de chaleur jusqu'à un moment où la température du corps de génération de chaleur atteint la température prédéterminée.

4. Instrument opératoire (1) selon la revendication 1, dans lequel l'unité (3) d'alimentation électrique est commandée de façon à ce qu'un parmi une tension constante ou un courant constant soit délivré pendant une période dans laquelle la puissance électrique sensiblement constante est délivrée au corps (23) de génération de chaleur.

5. Instrument opératoire (1) selon la revendication 1, dans lequel l'unité (3) d'alimentation électrique est adaptée à détecter la température du corps (23) de génération de chaleur à partir d'une valeur de résistance du corps de génération de chaleur.

6. Instrument opératoire (1) selon la revendication 1, dans lequel l'unité (3) d'alimentation électrique est adaptée à commander le corps de génération de chaleur à une température constante après que la température du corps (23) de génération de chaleur a atteint la température prédéterminée.

7. Instrument opératoire (1) selon la revendication 1, dans lequel l'unité (3) d'alimentation électrique est adaptée à appliquer une puissance électrique au corps (23) de génération de chaleur de façon intermittente après que la température du corps de génération de chaleur a atteint la température prédéterminée.

8. Instrument opératoire (1) selon la revendication 1, dans lequel le corps (23) de génération de chaleur est un élément de génération de chaleur comprenant une résistance à film mince ou une résistance à film épais ayant un coefficient de température comme un motif de génération de chaleur sur la surface de celui-ci.

9. Instrument opératoire (1) selon la revendication 1, dans lequel le corps (23) de génération de chaleur a un coefficient de température positif, et dans lequel l'unité (3) d'alimentation électrique est construite de façon à calculer la température du corps de génération de chaleur à partir de la résistance électrique du corps de génération de chaleur et, lorsque la résistance électrique du corps de génération de chaleur atteint une valeur prédéterminée, à déterminer que la température du corps de génération de chaleur a atteint la température prédéterminée et à stopper l'alimentation électrique du corps de génération de chaleur ou à commuter la commande sur une commande de température constante qui maintient la température du corps de génération de chaleur à la température prédéterminée.

10. Instrument opératoire (1) selon la revendication 1, dans lequel l'unité d'alimentation électrique est adaptée à déterminer que la pince (2) ne traite pas l'anatomie lorsque la quantité de changement d'un parmi une tension devant être appliquée au corps de génération de chaleur, un courant devant être délivré au corps de génération de chaleur, et une résistance électrique du corps de génération de chaleur excède une valeur prédéterminée, et à stopper l'alimentation électrique du corps de génération de chaleur.

**11.** Instrument opératoire (1) selon la revendication 1, dans lequel l'unité (3) d'alimentation électrique délivre un courant de surveillance au corps (23) de génération de chaleur avant de démarrer la sortie vers le corps de génération de chaleur ou après avoir démarré la sortie, calcule une valeur de tension devant être appliquée au corps de génération de chaleur sur la base de valeurs de tension et de courant du courant de surveillance et d'une valeur prédéfinie de puissance électrique, et démarre l'alimentation électrique du corps de génération de chaleur sur la base de la valeur de tension calculée.

**12.** Instrument opératoire (1) selon la revendication 1, dans lequel la pince (2) comprend une paire d'organes de préhension (13, 14) aptes à ouvrir et fermer la section (2a) de traitement sur l'extrémité distale et une section (2b) d'opération pour ouvrir et fermer les organes de préhension (13, 14) sur le côté proximal, et dans lequel le corps (23) de génération de chaleur est prévu sur au moins un des organes de préhension (13, 14) de la pince.

**13.** Instrument opératoire (1) selon la revendication 1, dans lequel l'unité (3) d'alimentation électrique comprend un circuit (34) de calcul pour délivrer un courant de surveillance au corps (23) de génération de chaleur avant de démarrer la sortie vers le corps de génération de chaleur ou après avoir démarré la sortie, calculer une valeur de tension devant être appliquée au corps (23) de génération de chaleur sur la base de valeurs de tension et de courant du courant de surveillance et d'une valeur prédéfinie de puissance électrique, et dans lequel l'instrument opératoire démarre l'alimentation électrique du corps (23) de génération de chaleur sur la base de la valeur de tension calculée, calculée par le circuit (34) de calcul.

Fig. 1

Fig. 2

13
25
23
24
2 a

27  26
14

**Fig. 3**

13
23  25

**Fig. 4**

14

26

Fig. 5

29

28
28b
28a
29

28
28b
28a

29

23A

23

23A

24

Fig. 6

Fig. 7

EP 1 582 165 B1

Fig. 8

Fig. 9

Fig. 10

Fig. 11

Fig. 12

Fig. 13

Fig. 14

Fig. 15

Fig. 16

Fig. 17

Fig. 18

Fig. 19

Fig. 20

Fig. 21

Fig. 22

Fig. 23

Fig. 24

Fig. 25

**Fig. 26**

**Fig. 27**

Fig. 28

Fig. 29

Fig. 30

Fig. 31

Fig. 32

Fig. 33

Fig. 34

Fig. 35

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2004071405 A
- JP 3349139 B
- WO 0112090 A
- WO 3349139 A
- JP 10286260 A
- US 2003171747 A